# NOUVEAU FASCICULE DE BREVET EUROPEEN

(11) **EP 0 876 325 B2**
(45) Date de publication et mention de la décision concernant l'opposition: **07.06.2006**
(45) Mention de la délivrance du brevet: 09.01.2002
(21) Numéro de dépôt: 96934974.5
(22) Date de dépôt: 25.10.1996
(51) Int. Cl.: C07C 69/88, C11B 9/00

(54) **COMPOSITIONS PARFUMANTES ET DE PRODUITS PARFUMES ET PRODUITS AINSI OBTENUS**
RIECHSTOFFKOMPOSITIONEN UND RIECHSTOFFPRODUKTE UND SO ERHALTENE PRODUKTE
SCENTING COMPOSITIONS AND SCENTED PRODUCTS, AND RESULTING PRODUCTS

(30) Priorité: 27.10.1995 FR 9512687
(43) Date de publication de la demande: 11.11.1998
(73) Titulaire: RHODIA CHIMIE, 92408 Courbevoie Cédex (FR)
(72) Inventeur: STORET, Isabelle, F-383001 Les Eparres (FR)
(74) Mandataire: Dutruc-Rosset, Marie-Claude
(86) Numéro de dépôt international: PCT/FR1996/001673
(87) Numéro de publication internationale: WO 1997/015547

(56) Documents cités:
- US-A- 3 076 017
- US-A- 3 962 148
- US-A- 4 461 309
- JOURNAL OF ORGANIC CHEMISTRY, vol. 54, no. 1, 6 Janvier 1989, EASTON US, pages 38-46, XP002006515 BARRY B.SNIDER: "Manganese(III)-Based Oxidative Free-Radical Cyclizations.Oxidative Cyclization and Aromatization of 3-Oxo-6-heptenoate Esters."
- S. Arctander, "Perfume and Flavour Chemicals", monographs 1906, 2119, Montclair, N.J. USA, 1969
- Document montrant utilisation antérieure et vente du para-méthyl crésotate, 1986
- F. Cola, "Le livre du parfumeur", Castermann, Paris, FR, 1931
- G. Salvadori, Perfumer and Flavour, 18, 20-21, 1993
- "The ABCs of Perfumery", publié dans Perfumer and Flavourist, 29, 2004

## Description

La présente invention a trait à des compositions parfumantes et de produits parfumés ainsi obtenus.

La présente invention vise l'emploi dans le domaine de la parfumerie de certains esters d'acide alkylsalicylique. Lesdits composés possèdent des propriétés olfactives intéressantes et peuvent être employés, entre autres, pour la préparation de compositions parfumantes et de produits parfumés.

L'industrie de la parfumerie est constamment à la recherche de produits qui par l'originalité, le volume et la puissance de leur fragrance puissent conférer aux compositions dans lesquelles elles interviennent, un caractère tout à fait particulier.

Il est mentionné dans l'ouvrage Perfume and Flavor Chemicals de Steffen ARCTANDER - Montclair N. J. (U.S.A) [1969] qu'il est possible d'utiliser en parfumerie le 2-hydroxy-4-méthylsalicylate d'amyle ou le 2-hydroxy-4-méthylsalicylate de méthyle. Toutefois, il est également mentionné que le paraméthyl méthylsalicylate (ou 2-hydroxy-4-méthylsalicylate de méthyle) présente une odeur faible « Its weaker odor and higher cost makes it rather uninteresting as compared to methylsalicylate ».

Il a maintenant été trouvé que les esters d'un acide alkylsalicylique définis ci-après, et plus particulièrement - des esters d'un acide méthylsalicylique présentaient des propriétés olfactives originales.

Il est à noter qu'il est impossible pour l'Homme du Métier de prévoir si un composé chimique donné possédera ou non, une odeur intéréssante du point de vue olfactif susceptible d'être retenue dans le domaine de la parfumerie.

Plus spécifiquement, l'invention réside dans l'utilisation comme ingrédient parfumant, d'un ester d'un acide alkylsalicylique choisi parmi :
- le 2-hydroxy-3-méthyl-benzoate de méthyle
- le 2-hydroxy-3-méthyl-benzoate d'éthyle
- le 2-hydroxy-3-méthyl-benzoate d'isopropyle
- le 2-hydroxy-3-méthyl-benzoate de n-propyle
- le 2-hydroxy-3-méthyl-benzoate de n-butyle
- le 2-hydroxy-3-méthyl-benzoate d'isobutyle
- le 2-hydroxy-3-méthyl-benzoate d'amyle
- le 2-hydroxy-3-méthyl-benzoate d'isoamyle
- le 2-hydroxy-3-méthyl-benzoate de n-hexyle
- le 2-hydroxy-3-méthyl-benzoate de 2-éthylhexyle
- le 2-hydroxy-3-méthyl-benzoate de cyclohexyle
- le 2-hydroxy-3-méthyl-benzoate de benzyle
- le 2-hydroxy-3-méthyl-benzoate de β-phényléthyle
- le 2-hydroxy-4-méthyl-benzoate d'éthyle
- le 2-hydroxy-5-méthyl-benzoate de méthyle
- le 2-hydroxy-5-méthyl-benzoate d'éthyle
- le 2-hydroxy-5-méthyl-benzoate d'isopropyle
- le 2-hydroxy-5-méthyl-benzoate de n-propyle
- le 2-hydroxy-5-méthyl-benzoate de n-butyle
- le 2-hydroxy-5-méthyl-benzoate d'isobutyle
- le 2-hydroxy-5-méthyl-benzoate d'amyle
- le 2-hydroxy-5-méthyl-benzoate d'isoamyle
- le 2-hydroxy-5-méthyl-benzoate de n-hexyle
- le 2-hydroxy-5-méthyl-benzoate de 2-éthylhexyle
- le 2-hydroxy-5-méthyl-benzoate de cyclohexyle
- le 2-hydroxy-5-méthyl-benzoate de benzyle
- le 2-hydroxy-5-méthyl-benzoate de β-phényléthyle.

La présente invention a également pour objet des compositions parfumantes, substances et produits parfumés caractérisés par le fait qu'ils comprennent, à titre de principe actif ayant une influence sur l'odeur, une quantité efficace d'un ester d'un acide alkylsalicylique choisi dans la liste donnée ci-dessus et désigné par la suite « ester d'un acide alkylsalicylique de l'invention ».

L'ingrédient parfumant, objet de l'invention est un ester d'un acide alkylsalicylique tel que précité.

Les esters d'alkysalicylique de l'invention exhalent une odeur très intéressante.

Ce sont des produits qui peuvent être utilisés, comme ingrédients parfumants, dans les compositions parfumantes, substances et produits parfumés.

Par "compositions parfumantes", on désigne des mélanges de divers ingrédients tels que solvants, supports solides ou liquides, fixateurs, composés odorants divers, etc..., dans lesquels sont incorporés les esters d'un acide alkylsalicylique de l'invention lesquels sont utilisés pour procurer à divers types de produits finis, la fragrance recherchée.

Les bases pour parfum constituent des exemples préférés de compositions parfumantes dans lesquelles les esters d'un acide alkylsalicylique de l'invention peuvent être avantageusement utilisés.

Les eaux de toilettes, les lotions après rasage, les parfums, les savons, les gels de bain ou de douche ou les produits déodorants ou antiperspirants, qu'ils soient sous forme de sticks ou de lotions constituent des exemples de substances ou de produits finis dans lesquels les esters d'un acide alkylsalicylique de l'invention apportent leur note originale.

Ils peuvent intervenir aussi dans les shampooings et dans les produits capillaires de tout type.

Ils peuvent aussi parfumer les talcs ou poudres de toute nature.

Ils peuvent également convenir pour les désodorisants d'air ambiant ou tout produit d'entretien.

Un autre exemple de compositions dans lesquelles lesdits composés peuvent être introduits de façon avantageuse, est représenté par les compositions détergentes usuelles. Ces compositions comprennent généralement un ou plusieurs des ingrédients suivants : agents tensio-actifs anioniques, cationiques ou amphotères, agents de blanchiment, azurants optiques, charges diverses, agents anti-redéposition. La nature de ces divers composants n'est pas critique et les esters d'un acide alkylsalicylique de de l'invention peuvent être ajoutés à tout type de composition détergente. Ils peuvent être introduits dans les adoucissants pour textiles, sous forme liquide ou dans les compositions déposées sur support, le plus souvent un non-tissé, destinées à être employées dans les sèche-linges.

La teneur des compositions selon l'invention en ester d'un acide alkylsalicylique de l'invention exprimée en pourcentage en poids dans la composition considérée dépend de la nature de ladite composition (base pour parfum ou eau de toilette par exemple) et de la puissance et de la nature de l'effet recherché au niveau produit final. Il va de soi que dans une base pour parfum, la teneur en ester d'un acide alkylsalicylique de l'invention peut être très importante, par exemple supérieure à 50 % en poids et peut atteindre 90 % en poids tandis que dans un parfum, une eau de toilette ou une lotion après rasage, cette teneur pourra être très inférieure à 50 % en poids.

La teneur en ester d'un acide alkylsalicylique peut être dans les compositions détergentes, notamment ménagères ou dans des savons, de l'ordre de 1 à 2%.

II peut également intervenir dans les shampooings parfumés à raison de 0,5 à 2 % ou pour parfumer tout produit capillaire.

Ainsi la limite inférieure de la teneur en ester d'un acide alkylsalicylique de l'invention peut être celle qui provoque une modification perceptible à l'odorat de la fragrance ou de la note du produit fini. Dans certains cas, cette teneur minimale peut être de l'ordre de 0,01 % en poids. On peut évidemment faire appel à des teneurs non comprises dans les limites des teneurs indiquées ci-avant sans pour autant sortir du cadre de la présente invention.

Une des voie d'accès aux esters d'un acide alkylsalicylique de l'invention consistent à faire réagir l'acide alkylsalicylique correspondant et un alcool, en présence d'un catalyseur acide.

Concernant les acides alkylsalicyliques correspondants, on fait appel plus particulièrement à l'acide 2-hydroxy-3-méthyl-benzoïque, l'acide 2-hydroxy-4-méthyl-benzoïque, l'acide 2-hydroxy-5-méthyl-benzoïque.

Comme exemples d'alcools, on met en oeuvre préférentiellement, les alcools primaires ou secondaires ayant de 1 à 12 atomes de carbone de préférence, le méthanol, l'éthanol, le cyclohexanol, l'alcool benzylique, l'alcool β-phényléthylique.

Conformément au procédé de l'invention, on fait réagir ledit acide avec un alcool.

On trouve dans le commerce les acides alkylsalicyliques.

On peut envisager plusieurs modes de préparation.

Une première variante consiste à faire réagir un acide alkylsalicylique avec l'alcool choisi.

Il est également possible d'effectuer l'estérification en présence d'un solvant organique. Le solvant organique est choisi de telle sorte qu'il forme un azéotrope avec l'eau et que le point d'ébullition de son azéotrope avec l'eau soit inférieur à celui de l'alcool engagé. Comme exemples de solvants, on peut citer notamment, le toluène, le cumène ou le pseudocumène.

D'une manière préférentielle, on choisit de faire appel à un procédé d'estérification directe, en l'absence de solvant organique, pour les alcools ayant de 1 à 5 atomes de carbone.

Dans le cas d'alcools lourds ayant plus de 5 atomes de carbone, il est préférable de conduire la réaction, en présence d'un solvant organique.

Les différentes réactions sont conduites en présence d'un catalyseur classique de type acide. On peut citer notamment, l'acide sulfurique, l'acide chlorhydrique, l'acide p-toluène sulfonique, les titanates d'alcoyle, de préférence le titanate d'isopropyle ou de n-butyle, l'oxyde d'antimoine.

La quantité des réactifs en présence est déterminée de telle sorte que l'alcool soit généralement en excès par rapport à l'acide alkylsalicylique. L'excès varie largement, de préférence de 50 à 3000 % par rapport à la quantité stoechiométrique. Il est choisi plus préférentiellement entre 100 et 2000 % de la quantité stoechiométrique.

La quantité de catalyseur utilisé, exprimée par rapport au poids de l'acide alkylsalicylique, est choisie avantageusement entre 1 et 30 %.

Lorsqu'il y a présence d'un solvant organique, la quantité mise en jeu peut être très variable. A titre indicatif, on peut préciser que la quantité de solvant organique peut représenter de 50 à 1000 % du poids de l'acide alkylsalicylique mis en jeu.

La température de la réaction est choisie de manière qu'elle soit suffisante pour permettre l'accomplissement de la réaction.

La température de la réaction est choisie de préférence entre 50 et 150°C.

La réaction est conduite avantageusement sous pression atmosphérique.

On conduit la réaction de préférence sous atmosphère d'un gaz inerte qui peut être l'azote ou un gaz rare, de préférence l'argon.

D'un point de vue pratique, le procédé selon l'invention est simple à mettre en oeuvre.

Les différents réactifs peuvent être introduits dans n'importe quel ordre. D'une manière préférée, on choisit l'ordre des réactifs suivants : on introduit l'acide alkylsalicylique et l'alcool puis le catalyseur acide.

On porte le milieu réactionnel à la température désirée, tout en maintenant le milieu réactionnel sous agitation.

Au cours de la réaction, il y a formation d'eau dans le milieu réactionnel. Une variante préférée de l'invention consiste à l'éliminer du milieu réactionnel, au fur et à mesure de leur formation, par tout moyen connu, notamment par distillation azéotropique.

En fin de réaction, on obtient l'ester d'un acide alkylsalicylique *de l'invention,* l'alcool en excès et le catalyseur.

On peut récupérer l'ester d'un acide alkylsalicylique formé à partir du milieu réactionnel, par tout moyen approprié.

Ainsi, on peut notamment faire un lavage à l'eau suivie d'une neutralisation à l'aide d'une base.

La quantité de base, de préférence la soude, le carbonate ou le bicarbonate de sodium est telle que le pH soit compris entre 6 et 8.

On sépare la phase organique que l'on fractionne par distillation. On recueille le plus souvent, d'abord l'excès d'alcool puis l'ester d'un acide alkylsalicylique de l'invention.

On donne ci-après des exemples de réalisation de l'invention.

### Exemple 1

1 - Dans cet exemple, on réalise la préparation du 2-hydroxy-4-méthylbenzoate d'éthyle.

Dans un réacteur, on additionne 50 g d'acide 2-hydroxy-4-méthylbenzoïque à 250 ml d'éthanol. Par l'intermédiaire d'une ampoule à brome, on introduit goutte à goutte, 40 ml d'acide sulfurique ; on refroidit s'il y a ébullition de l'alcool.

On chauffe 3 heures à reflux en agitant.

Puis on refroidit à température ambiante, on coule sur environ 100 ml d'eau glacée, et on évapore l'alcool sous pression réduite.

On procède à trois extractions de la phase aqueuse par de l'éther éthylique.

Les phases organiques combinées sont lavées par une solution saturée d'hydrogénocarbonate de sodium jusqu'à pH neutre, puis lavées une fois avec de l'eau de façon à éliminer les sels.

On sèche la phase organique sur sulfate de magnésium et on évapore sous pression réduite (200 mm de mercure/ 2,66.10⁴ Pa) pour obtenir l'ester brut.

L'ester éthylique est distillé à 82°C, sous pression réduite de 1 mm de mercure (133 Pa), pour fournir le 2-hydroxy-4-méthylbenzoate d'éthyle d'une pureté supérieure à 97 %.
2- L'odeur du 2-hydroxy-4-méthylbenzoate d'éthyle est une odeur fleurie animale typique des notes Ylang.

### Exemple 2*

1 - Dans cet exemple, on décrit la préparation du 2-hydroxy-4-méthylbenzoate de n-hexyle qui est reproduite dans d'autres exemples.

Dans un réacteur muni d'un condenseur "Dean-Stark", on additionne 59 g d'acide 2-hydroxy-4-méthylbenzoïque et 41 g de n-hexanol sec à 150 ml de toluène. On ajoute enfin 5 % d'acide p-toluène sulfonique, qui joue le rôle de catalyseur acide.

On porte à reflux pendant 24 heures pendant lesquelles on peut observer l'élimination d'eau.

On ramène à température ambiante et on lave la phase organique par une solution saturée d'hydrogénocarbonate de sodium jusqu'à pH neutre.

Puis on lave la phase organique par de l'eau avant de la sécher sur sulfate de magnésium. On évapore sous pression réduite le toluène et une partie du n-hexanol.

L'ester brut est distillé, sous pression réduite de 2 mm de mercure (2,66 Pa), pour fournir le 2-hydroxy-4-méthylbenzoate de n-hexyle d'une pureté supérieure à 97% .
2 - L'odeur du 2-hydroxy-4-méthylbenzoate de n-hexyle est une odeur terpénique de mousse de chêne.

### Exemple 3*

1 - Dans cet exemple, on décrit la préparation du 2-hydroxy-4-méthylbenzoate d'isopropyle qui est reproduite dans d'autres exemples.

Dans un réacteur, on fait barboter de l'acide chlorhydrique gazeux dans 250 ml d'isopropanol sec jusqu'à l'obtention d'une solution à 2 % en poids d'HCl.

On additionne ensuite 25 g d'acide 2-hydroxy-4-méthylbenzoïque et on chauffe à reflux pendant 24 h.

On évapore l'alcool et on reprend le résidu avec une solution aqueuse saturée en hydrogénocarbonate de sodium.

On procède à 3 extractions de la phase aqueuse par de l'éther éthylique.

Les phases organiques combinées sont lavées par de l'eau, séchées sur sulfate de magnésium puis évaporées.

L'ester brut est distillé sous pression réduite de 2 mm de mercure (2,66 Pa).
2- L'odeur du 2-hydroxy-4-méthylbenzoate d'isopropyle est une odeur de céleri.

### Exemple 4

1 - Dans cet exemple, on prépare le 2-hydroxy-3-méthylbenzoate de méthyle selon le mode opératoire décrit dans l'exemple 1.
2 - L'odeur du 2-hydroxy-3-méthylbenzoate de méthyle est une odeur cuirée.

### Exemple 5

1 - Dans cet exemple, on prépare le 2-hydroxy-3-méthylbenzoate d'isoamyle selon le mode opératoire décrit dans l'exemple 3.
2 - L'odeur du 2-hydroxy-3-méthylbenzoate d'isoamyle est une odeur fruitée cuir.

### Exemple 6

1 - Dans cet exemple, on prépare le 2-hydroxy-3-méthylbenzoate de n-hexyle selon le mode opératoire décrit dans l'exemple 2.
2 - L'odeur du 2-hydroxy-3-méthylbenzoate de n-hexyle est une odeur fruitée.

### Exemple 7

1 - Dans cet exemple, on prépare le 2-hydroxy-3-méthylbenzoate d'isopropyle selon le mode opératoire décrit dans l'exemple 3.
2- L'odeur du 2-hydroxy-3-méthylbenzoate d'isopropyle est une odeur cuirée.

### Exemple 8

1 - Dans cet exemple, on prépare le 2-hydroxy-3-méthylbenzoate d'éthyle selon le mode opératoire décrit dans l'exemple 1.
2 - L'odeur du 2-hydroxy-3-méthylbenzoate d'éthyle est une odeur cuirée.

### Exemple 9

1 - Dans cet exemple, on prépare le 2-hydroxy-5-méthylbenzoate de méthyle selon le mode opératoire décrit dans l'exemple 1.
2- L'odeur du 2-hydroxy-5-méthylbenzoate de méthyle est une odeur de violette safranée.

### Exemple 10

1 - Dans cet exemple, on prépare le 2-hydroxy-5-méthylbenzoate d'éthyle selon le mode opératoire décrit dans l'exemple 1.
2 - L'odeur du 2-hydroxy-5-méthylbenzoate d'éthyle est une odeur d'aubépine.

### Exemple 11

1 - Dans cet exemple, on prépare le 2-hydroxy-5-méthylbenzoate de propyle selon le mode opératoire décrit dans l'exemple 1.
2- L'odeur du 2-hydroxy-5-méthylbenzoate de propyle est une odeur d'aubépine.

### Exemple 12

1 - Dans cet exemple, on prépare le 2-hydroxy-5-méthylbenzoate de butyle selon le mode opératoire décrit dans l'exemple 1.
2 - L'odeur du 2-hydroxy-5-méthylbenzoate de butyle est une odeur d'aubépine.

### Exemple 13

1 - Dans cet exemple, on prépare le 2-hydroxy-5-méthylbenzoate d'amyle selon le mode opératoire décrit dans l'exemple 1.
2 - L'odeur du 2-hydroxy-5-méthylbenzoate d'amyle est une odeur d'aubépine.

### Exemple 14

1 - Dans cet exemple, on prépare le 2-hydroxy-5-méthylbenzoate de n-hexyle selon le mode opératoire décrit dans l'exemple 2.
2- L'odeur du 2-hydroxy-5-méthylbenzoate de n-hexyle est une odeur d'aubépine.

### Exemple 15

1 - Dans cet exemple, on prépare le 2-hydroxy-5-méthylbenzoate de benzyle selon le mode opératoire décrit dans l'exemple 2.
2 - L'odeur du 2-hydroxy-5-méthylbenzoate de benzyle est une odeur d'aubépine.

### Exemple 16

1 - Dans cet exemple, on prépare le 2-hydroxy-5-méthylbenzoate de cyclohexyle selon le mode opératoire décrit dans l'exemple 3.
2 - L'odeur du 2-hydroxy-5-méthylbenzoate de cyclohexyle est une odeur d'aubépine.

### Exemple 17

1 - Dans cet exemple, on prépare le 2-hydroxy-5-méthylbenzoate d'isopropyle selon le mode opératoire décrit dans l'exemple 3.
2- L'odeur du 2-hydroxy-5-méthylbenzoate d'isopropyle est une odeur d'aubépine.

### Exemple 18

1 - Dans cet exemple, on prépare le 2-hydroxy-5-méthylbenzoate d'isobutyle selon le mode opératoire décrit dans l'exemple 3.
2- L'odeur du 2-hydroxy-5-méthylbenzoate d'isobutyle est une odeur d'aubépine.

### Exemple 19

1 - Dans cet exemple, on prépare le 2-hydroxy-5-méthylbenzoate d'isoamyle selon le mode opératoire décrit dans l'exemple 3.
2 - L'odeur du 2-hydroxy-5-méthylbenzoate d'isoamyle est une odeur d'aubépine.

### Exemple 20

On donne ci-après un exemple de mis en oeuvre du 2-hydroxy-4-méthylbenzoate d'éthyle dans une composition parfumante sous forme liquide :
10% de 2-hydroxy-4-méthylbenzoate d'éthyle
10% de salicylate de benzyle
10% d'hydroxycitronellal
10% d'anthranillate de méthyle
2% d'isoeugénol
4% de gamma nonalactone
54% d'aldéhyde alpha hexylcinnamique.

## Revendications

1. Utilisation comme ingrédient parfumant, d'un ester d'un acide alkylsalicylique choisi parmi :
- le 2-hydroxy-3-méthyl-benzoate de méthyle
- le 2-hydroxy-3-méthyl-benzoate d'éthyle
- le 2-hydroxy-3-méthyl-benzoate d'isopropyle
- le 2-hydroxy-3-méthyl-benzoate de n-propyle
- le 2-hydroxy-3-méthyl-benzoate de n-butyle
- le 2-hydroxy-3-méthyl-benzoate d'isobutyle
- le 2-hydroxy-3-méthyl-benzoate d'amyle
- le 2-hydroxy-3-méthyl-benzoate d'isoamyle
- le 2-hydroxy-3-méthyl-benzoate de n-hexyle
- le 2-hydroxy-3-méthyl-benzoate de 2-éthylhexyle
- le 2-hydroxy-3-méthyl-benzoate de cyclohexyle
- le 2-hydroxy-3-méthyl-benzoate de benzyle
- le 2-hydroxy-3-méthyl-benzoate de β-phényléthyle
- le 2-hydroxy-4-méthyl-benzoate d'éthyle
- le 2-hydroxy-5-méthyl-benzoate de méthyle
- le 2-hydroxy-5-méthyl-benzoate d'éthyle
- le 2-hydroxy-5-méthyl-benzoate d'isopropyle
- le 2-hydroxy-5-méthyl-benzoate de n-propyle
- le 2-hydroxy-5-méthyl-benzoate de n-butyle
- le 2-hydroxy-5-méthyl-benzoate d'isobutyle
- le 2-hydroxy-5-méthyl-benzoate d'amyle
- le 2-hydroxy-5-méthyl-benzoate d'isoamyle
- le 2-hydroxy-5-méthyl-benzoate de n-hexyle
- le 2-hydroxy-5-méthyl-benzoate de 2-éthylhexyle
- le 2-hydroxy-5-méthyl-benzoate de cyclohexyle
- le 2-hydroxy-5-méthyl-benzoate de benzyle
- le 2-hydroxy-5-méthyl-benzoate de β-phényléthyle.

2. Utilisation selon la revendication 1 dans le domaine de la parfumerie, à titre d'odeur fleurie animale typique des notes Ylang, du 2-hydroxy-4-méthylbenzoate d'éthyle.

3. Utilisation selon la revendication 1 dans le domaine de la parfumerie, à titre d'odeur de violette safranée, du 2-hydroxy-5-méthylbenzoate de méthyle.

4. Utilisation selon la revendication 1 dans le domaine de la parfumerie, à titre d'odeur cuirée, du 2-hydroxy-3-méthyl-benzoate de méthyle, du 2-hydroxy-3-méthyl-benzoate d'éthyle, ou du 2-hydroxy-3-méthyl-benzoate d'isopropyle.

5. Utilisation selon la revendication 1 dans le domaine de la parfumerie, à titre d'odeur fruitée cuir, du 2-hydroxy-3-méthyl-benzoate d'isoamyle.

6. Utilisation selon la revendication 1 dans le domaine de la parfumerie, à titre d'odeur fruitée, du 2-hydroxy-3-méthyl-benzoate de n-hexyle.

7. Utilisation selon la revendication 1 dans le domaine de la parfumerie, à titre d'odeur d'aubépine, du 2-hydroxy-5-méthyl-benzoate d'éthyle, du 2-hydroxy-5-méthyl-benzoate d'isopropyle, du 2-hydroxy-5-méthyl-benzoate de n-propyle, du 2-hydroxy-5-méthyl-benzoate de n-butyle, du 2-hydroxy-5-méthyl-benzoate d'isobutyle, du 2-hydroxy-5-méthyl-benzoate d'amyle, du 2-hydroxy-5-méthyl-benzoate d'isoamyle, du 2-hydroxy-5-méthyl-benzoate de n-hexyle, du 2-hydroxy-5-méthyl-benzoate de cyclohexyle, du 2-hydroxy-5-méthyl-benzoate de benzyle.

8. Compositions parfumantes, substances et produits parfumés **caractérisés par le fait qu'**ils comprennent, à titre de principe actif ayant une influence sur l'odeur, une quantité efficace d'un ester d'un acide alkylsalicylique choisi parmi:
- le 2-hydroxy-3-méthyl-benzoate de méthyle
- le 2-hydroxy-3-méthyl-benzoate d'éthyle
- le 2-hydroxy-3-méthyl-benzoate d'isopropyle
- le 2-hydroxy-3-méthyl-benzoate de n-propyle
- le 2-hydroxy-3-méthyl-benzoate de n-butyle
- le 2-hydroxy-3-méthyl-benzoate d'isobutyle
- le 2-hydroxy-3-méthyl-benzoate d'amyle
- le 2-hydroxy-3-méthyl-benzoate d'isoamyle
- le 2-hydroxy-3-méthyl-benzoate de n-hexyle
- le 2-hydroxy-3-méthyl-benzoate de 2-éthylhexyle
- le 2-hydroxy-3-méthyl-benzoate de cyclohexyle
- le 2-hydroxy-3-méthyl-benzoate de benzyle
- le 2-hydroxy-3-méthyl-benzoate de β-phényléthyle
- le 2-hydroxy-4-méthyl-benzoate d'éthyle
- le 2-hydroxy-5-méthyl-benzoate de méthyle
- le 2-hydroxy-5-méthyl-benzoate d'éthyle
- le 2-hydroxy-5-méthyl-benzoate d'isopropyle
- le 2-hydroxy-5-méthyl-benzoate de n-propyle
- le 2-hydroxy-5-méthyl-benzoate de n-butyle
- le 2-hydroxy-5-méthyl-benzoate d'isobutyle
- le 2-hydroxy-5-méthyl-benzoate d'amyle
- le 2-hydroxy-5-méthyl-benzoate d'isoamyle
- le 2-hydroxy-5-méthyl-benzoate de n-hexyle
- le 2-hydroxy-5-méthyl-benzoate de 2-éthylhexyle
- le 2-hydroxy-5-méthyl-benzoate de cyclohexyle
- le 2-hydroxy-5-méthyl-benzoate de benzyle
- le 2-hydroxy-5-méthyl-benzoate de β-phényléthyle.

9. Article parfumé selon la revendication 8 sous forme de parfum, d'eau de toilette, de lotions après rasage, de parfums, de savons, de gels de bain ou de douche, de produits déodorants ou antiperspirants, de shampooings ou tout produit capillaire, de talcs ou poudres de toute nature, de désodorisants d'air ambiant, de tout produit d'entretien ou de compositions détergentes, d'adoucissants pour textiles.

## Claims

1. Use, as a perfuming ingredient, of an alkylsalicylic acid ester selected from:
• methyl 2-hydroxy-3-methyl-benzoate;
• ethyl 2-hydroxy-3-methyl-benzoate;
• isopropyl 2-hydroxy-3-methyl-benzoate;
• n-propyl 2-hydroxy-3-methyl-benzoate;
• n-butyl 2-hydroxy-3-methyl-benzoate;
• isobutyl 2-hydroxy-3-methyl-benzoate;
• amyl 2-hydroxy-3-methyl-benzoate;
• isoamyl 2-hydroxy-3-methyl-benzoate;
• n-hexyl 2-hydroxy-3-methyl-benzoate;
• 2-ethylhexyl 2-hydroxy-3-methyl-benzoate;
• cyclohexyl 2-hydroxy-3-methyl-benzoate;
• benzyl 2-hydroxy-3-methyl-benzoate;
• β-phenylethyl 2-hydroxy-3-methyl-benzoate;
• ethyl 2-hydroxy-4-methyl-benzoate;
• methyl 2-hydroxy-5-methyl-benzoate;
• ethyl 2-hydroxy-5-methyl-benzoate;
• isopropyl 2-hydroxy-5-methyl-benzoate;
• n-propyl 2-hydroxy-5-methyl-benzoate;
• n-butyl 2-hydroxy-5-methyl-benzoate;
• isobutyl 2-hydroxy-5-methyl-benzoate;
• amyl 2-hydroxy-5-methyl-benzoate;
• isoamyl 2-hydroxy-5-methyl-benzoate;
• n-hexyl 2-hydroxy-5-methyl-benzoate;
• 2-ethylhexyl 2-hydroxy-5-methyl-benzoate;
• cyclohexyl 2-hydroxy-5-methyl-benzoate;
• benzyl 2-hydroxy-5-methyl-benzoate;
• β-phenylethyl 2-hydroxy-5-methyl-benzoate.

2. Use in the perfumery field, in accordance with claim 1, of ethyl 2-hydroxy-4-methylbenzoate, as an animal flowery odour typical ofYlang notes.

3. Use in the perfumery field, in accordance with claim 1, of methyl 2-hydroxy-5-methyl-benzoate, as a saffron violet odour.

4. Use in the perfumery field, in accordance with claim 1,of methyl 2-hydroxy-3-methyl-benzoate, ethyl 2-hydroxy-3-methyl-benzoate or isopropyl 2-hydroxy-3-methyl-benzoate, as a leathery odour.

5. Use in the perfumery field, in accordance with claim 1, of isoamyl 2-hydroxy-3-methyl-benzoate, as a fruity leathery odour.

6. Use in the perfumery field, in accordance with claim 1, of n-hexyl 2-hydroxy-3-methyl-benzoate, as a fruity odour.

7. Use in the perfumery field, in accordance with claim 1, of ethyl 2-hydroxy-5-methylbenzoate, isopropyl 2-hydroxy-5-methyl-benzoate, n-propyl 2-hydroxy-5-methylbenzoate, n-butyl 2-hydroxy-5-methyl-benzoate, isobutyl 2-hydroxy-5-methylbenzoate, amyl 2-hydroxy-5-methyl-benzoate, isoamyl 2-hydroxy-5-methyl-benzoate, n-hexyl 2-hydroxy-5-methyl-benzoate, cyclohexyl 2-hydroxy-5-methyl-benzoate, benzyl 2-hydroxy-5-methyl-benzoate, as a may-blossom odour.

8. Perfuming compositions, perfumed products and substances, **characterized in that** they comprise, as the active principle having an influence on the odour, an effective quantity of an alkylsalicylic acid ester selected from:
• methyl 2-hydroxy-3-methyl-benzoate;
• ethyl 2-hydroxy-3-methyl-benzoate;
• isopropyl 2-hydroxy-3-methyl-benzoate;
• n-propyl 2-hydroxy-3-methyl-benzoate;
• n-butyl 2-hydroxy-3-methyl-benzoate;
• isobutyl 2-hydroxy-3-methyl-benzoate;
• amyl 2-hydroxy-3-methyl-benzoate;
• isoamyl 2-hydroxy-3-methyl-benzoate;
• n-hexyl 2-hydroxy-3-methyl-benzoate;
• 2-ethylhexyl 2-hydroxy-3-methyl-benzoate;
• cyclohexyl 2-hydroxy-3-methyl-benzoate;
• benzyl 2-hydroxy-3-methyl-benzoate;
• β-phenylethyl 2-hydroxy-3-methyl-benzoate;
• ethyl 2-hydroxy-4-methyl-benzoate;
• methyl 2-hydroxy-5-methyl-benzoate;
• ethyl 2-hydroxy-5-methyl-benzoate;
• isopropyl 2-hydroxy-5-methyl-benzoate;
• n-propyl 2-hydroxy-5-methyl-benzoate;
• n-butyl 2-hydroxy-5-methyl-benzoate;
• isobutyl 2-hydroxy-5-methyl-benzoate;
• amyl 2-hydroxy-5-methyl-benzoate;
• isoamyl 2-hydroxy-5-methyl-benzoate;
• n-hexyl 2-hydroxy-5-methyl-benzoate;
• 2-ethylhexyl 2-hydroxy-5-methyl-benzoate;
• cyclohexyl 2-hydroxy-5-methyl-benzoate;
• benzyl 2-hydroxy-5-methyl-benzoate;
• β-phenylethyl 2-hydroxy-5-methyl-benzoate.

9. A perfumed article according to claim 8 in the form of a perfume, eau de toilette, after-shave lotion, perfume, soap, bath or shower gel, deodorising or antiperspirant product, shampoo or any other hair-care product, talc or powder of any type, an air freshener, any cleaning product or detergent composition, or a fabric softener.

## Patentansprüche

1. Verwendung eines Alkylsalicylsäureesters als Riechstoffbestandteil bzw. duftvermittelnder Inhaltsstoff, ausgewählt aus den folgenden Verbindungen:
- 2-Hydroxy-3-methylbenzoesäuremethylester,
- 2-Hydroxy-3-methylbenzoesäureethylester,
- 2-Hydroxy-3-methylbenzoesäureisopropylester,
- 2-Hydroxy-3-methylbenzoesäure-n-propylester,
- 2-Hydroxy-3-methylbenzoesäure-n-butylester,
- 2-Hydroxy-3-methylbenzoesäureisobutylester,
- 2-Hydroxy-3-methylbenzoesäureamylester,
- 2-Hydroxy-3-methylbenzoesäureisoamylester,
- 2-Hydroxy-3-methylbenzoesäure-2-hexylester,
- 2-Hydroxy-3-methylbenzoesäure-2-ethylhexylester,
- 2-Hydroxy-3-methylbenzoesäurecyclohexylester,
- 2-Hydroxy-3-methylbenzoesäurebenzylester,
- 2-Hydroxy-3-methylbenzoesäure-β-phenylethylester,
- 2-Hydroxy-4-methylbenzoesäureethylester,
- 2-Hydroxy-5-methylbenzoesäuremethylester,
- 2-Hydroxy-5-methylbenzoesäureethylester,
- 2-Hydroxy-5-methylbenzoesäureisopropylester,
- 2-Hydroxy-5-rriethylbenzoesäure-n-propylester,
- 2-Hydroxy-5-methylbenzoesäure-n-butylester,
- 2-Hydroxy-5-methylbenzoesäureisobutylester,
- 2-Hydroxy-5-methylbenzoesäureamylester,
- 2-Hydroxy-5-methylbenzoesäureisoamylester,
- 2-Hydroxy-5-methylbenzoesäure-n-hexylester,
- 2-Hydroxy-5-methylbenzoesäure-2-ethylhexylester,
- 2-Hydroxy-5-methylbenzoesäurecyclohexylester,
- 2-Hydroxy-5-methylbenzoesäurebenzylester und
- 2-Hydroxy-5-methylbenzoesäure-β-phenylethylester.

2. Verwendung nach Anspruch 1 von 2-Hydroxy-4-methylbenoesäureethylester im Bereich von Parfümwaren bzw. Kosmetikartikeln als für die Noten Ylang typischer tierisch-blumiger Duft.

3. Verwendung nach Anspruch 1 von 2-Hydroxy-5-methylbenzoesäuremethylester im Bereich von Parfümwaren bzw. Kosmetikartikeln als Duft von Krokusveilchen.

4. Verwendung nach Anspruch 1 von 2-Hydroxy-3-methylbenzoesäuremethylester, 2-Hydroxy-3-methylbenzoesäureethylester oder 2-Hydroxy-3-methylbenzoesäureisopropylester im Bereich von Parfümwaren bzw. Kosmetikartikeln als ledemer Duft.

5. Verwendung nach Anspruch 1 von 2-Hydroxy-3-methylbenzoesäureisoamylester im Bereich von Parfümwaren bzw. Kosmetikartikeln als fruchtiger Lederduft.

6. Verwendung nach Anspruch 1 von 2-Hydroxy-3-methylbenzoesäure-n-hexylester im Bereich von Parfümwaren bzw. Kosmetikartikeln als fruchtiger Duft.

7. Verwendung nach Anspruch 1 von 2-Hydroxy-5-methylbenzoesäureethylester, 2-Hydroxy-5-methylbenzoesäureisopropylester, 2-Hydroxy-5-methylbenzoesäure-n-propylester, 2-Hydroxy-5-methylbenzoesäure-n-butylester, 2-Hydroxy-5-methylbenzoesäureisobutylester, 2-Hydroxy-5-methylbenzoesäureamylester, 2-Hydroxy-5-methylbenzoesäureisoamylester, 2-Hydroxy-5-methylbenzoesäure-n-hexylester, 2-Hydroxy-5-methylbenzoesäurecyclohexylester oder 2-Hydroxy-5-methylbenzoesäurebenzylester im Bereich von Parfümwaren bzw. Kosmetikartikeln als Weißdornduft.

8. Riechstoffzusammensetzungen und Riechstoffsubstanzen und -produkte, **dadurch gekennzeichnet, daß** sie als Wirkstoff mit Einfluß auf den Duft eine wirksame Menge eines Alkylsäuresalicylsäureesters enthalten, ausgewählt aus den folgenden Verbindungen:
- 2-Hydroxy-3-methylbenzoesäuremethylester,
- 2-Hydroxy-3-methylbenzoesäureethylester,
- 2-Hydroxy-3-methylbenzoesäureisopropylester,
- 2-Hydroxy-3-methylbenzoesäure-n-propylester,
- 2-Hydroxy-3-methylbenzoesäure-n-butylester,
- 2-Hydroxy-3-methylbenzoesäureisobutylester,
- 2-Hydroxy-3-methylbenzoesäureamylester,
- 2-Hydroxy-3-methylbenzoesäureisoamylester,
- 2-Hydroxy-3-methylbenzoesäure-2-hexylester,
- 2-Hydroxy-3-methylbenzoesäure-2-ethylhexylester,
- 2-Hydroxy-3-methylbenzoesäurecyclohexylester,
- 2-Hydroxy-3-methylbenzoesäurebenzylester,
- 2-Hydroxy-3-methylbenzoesäure-β-phenylethylester,
- 2-Hydroxy-4-methylbenzoesäureethylester,
- 2-Hydroxy-5-methylbenzoesäuremethylester,
- 2-Hydroxy-5-methylbenzoesäureethylester,
- 2-Hydroxy-5-methylbenzoesäureisopropylester,
- 2-Hydroxy-5-methylbenzoesäure-n-propylester,
- 2-Hydroxy-5-methylbenzoesäure-n-butylester,
- 2-Hydroxy-5-methylbenzoesäureisobutylester,
- 2-Hydroxy-5-methylbenzoesäureamylester,
- 2-Hydroxy-5-methylbenzoesäureisoamylester,
- 2-Hydroxy-5-methylbenzoesäure-n-hexylester,
- 2-Hydroxy-5-methylbenzoesäure-2-ethylhexylester,
- 2-Hydroxy-5-methylbenzoesäurecyclohexylester,
- 2-Hydroxy-5-methylbenzoesäurebenzylester und
- 2-Hydroxy-5-methylbenzoesäure-β-phenylethylester.

9. Parfümerieware bzw. Kosmetikartikel nach Anspruch 8 in Form eines Parfüms, eines Eau-de-Toilette, von Aftershave-Lotionen, Düften (Duftstoffen), Seifen, Bade- oder Dusch-Gels, desodorierenden oder schweißhemmenden Produkten, Shampoos oder Haarprodukten aller Art, Körperpudern (Talkumpudern) oder Pudern aller Art, Raumluftdesodorantien, Pflegeprodukten aller Art, Reinigungszusammensetzungen oder Weichspülmitteln für Textilien.
